# EUROPÄISCHE PATENTSCHRIFT

(11) **EP 3 664 779 B1**
(45) Veröffentlichungstag und Bekanntmachung des Hinweises auf die Patenterteilung: **24.11.2021**
(21) Anmeldenummer: 18765011.4
(22) Anmeldetag: 13.08.2018
(51) Int. Cl.: A61K 9/00, A61B 5/00, A61M 37/00, A61M 25/00

(54) **MIKRONADELARRAY AUFWEISEND EINEN FARBUMSCHLAG-INDIKATOR**
MICRO-NEEDLE ARRAY COMPRISING A COLOR CHANGE INDICATOR
MATRICE DE MICRO-AIGUILLES PRÉSENTANT UN INDICATEUR DE CHANGEMENT DE COULEUR

(30) Priorität: 11.08.2017 DE 102017118419
(43) Veröffentlichungstag der Anmeldung: 17.06.2020
(73) Patentinhaber: LTS Lohmann Therapie-Systeme AG, 56626 Andernach (DE)
(72) Erfinder: HENNING, Andreas, 72469 Meßstetten (DE); MARX, Simone, 56076 Koblenz (DE); FLORIN, Larissa, 56317 Urbach (DE)
(74) Vertreter: Simandi, Claus
(86) Internationale Anmeldenummer: PCT/EP2018/071957
(87) Internationale Veröffentlichungsnummer: WO 2019/030417

(56) Entgegenhaltungen:
- EP-A1- 1 281 352
- WO-A1-2008/150829
- WO-A1-2009/081122
- WO-A1-2013/053022
- WO-A2-2012/153266
- US-A1- 2011 224 515

## Beschreibung

Die Erfindung betrifft einen Mikronadelarray zur Verwendung in der intradermalen Applikation umfassend eine Vielzahl an Mikronadeln an einem Träger, wobei dieser Mikronadelarray zur Hautpenetration an Mensch oder Tier geeignet ist und mindestens einen Farbumschlag-Indikator umfasst.

Mikronadelsysteme und Vorrichtungen bei denen Mikronadelarrays zur schmerzfreien intradermalen (bzw. transdermalen) Verabreichung von Stoffen, insbesondere Arzneimitteln verwendet werden, sind im Stand der Technik bekannt.

Die Haut besteht aus mehreren Schichten. Die äußerste Hautschicht, das Stratum Corneum, weist bekannte Barriereeigenschaften auf, um ein Eindringen von Fremdsubstanzen in den Körper und ein Austreten von Eigensubstanzen aus dem Körper zu verhindern. Das Stratum Corneum, das eine komplexe Struktur aus kompaktierten keratotischen Zellresten mit einer Dicke von ungefähr 10-30 Mikrometern ist, bildet hierzu eine wasserdichte Membran zum Schutz des Körpers aus. Diese natürliche Undurchlässigkeit des Stratum Corneum verhindert das Verabreichen der meisten pharmazeutischen Mittel und anderer Substanzen durch die Haut im Rahmen einer intradermalen Applikation.

Das Verabreichen verschiedener Substanzen erfolgt daher beispielsweise durch Erzeugen von Mikroporen oder Schnitten im Stratum Corneum und Zuführen bzw. Applikation eines Arzneimittels in oder unter das Stratum Corneum. Auf diese Weise können zahlreiche Arzneimittel z.B. auch subkutan oder intradermal bzw. intrakutan verabreicht werden.

Im Stand der Technik ist es nach wie vor schwierig festzustellen, ob ein Mikronadelarray ausreichend in die Haut penetriert ist und das Stratum Corneum erreicht bzw. als Barriere überwunden wird.

Im Stand der Technik beschreibt die WO 2009/081122 A1 ein Monitoring-System für die Arzneimittelfreisetzung über Mikronadeln. Das System ist jedoch nicht in der Lage, eine Penetration in das Stratum Corneum nachzuweisen, da der beigefügte Farbstoff lediglich eine Aussage dazu macht, ob ein Austreten eines Stoffes - hier Arzneistoff - aus den Mikronadeln erfolgt ist.

Ferner beschreibt die WO 2009/081122 A1 nicht den Einsatz von geeigneten Farbumschlag-Indikatoren, insbesondere pH-Indikatoren, insbesondere im pH-Bereich zwischen 4 und 8.

DE 10 2014 201 605 A1 beschreibt zwar das Problem, dass mittels eines Mikronadelarrays keine ausreichende Hautpenetration erfolgen kann und schlägt hierzu vor, dass Farbstoffe in Mikrokapseln in einem Applikationssystem vorliegen. Nachteilig ist jedoch, dass solche Mikrokapseln einen geeigneten Druck benötigen, so dass diese aufplatzen können und der Farbstoff austreten kann. Das Platzen der Mikrokapseln ist folglich unabhängig von der eigentlichen Hautpenetration und bedarf eines ausgeübten Mindestdruckes. Der Nachweis ist daher indirekt und mittelbar.

WO 2013/053022 A1 beschreibt polymere Arrays mit Projektionen im Mikrometerbereich zur Verabreichung von medizinischen Wirkstoffen mit Hilfe von Farbstoffindikatoren. Allerdings werden die Mikronadeln lediglich von dem Farbstoffindikator umhüllt.

An einen solchen dermalen Penetrationsindikator besteht die grundsätzliche Anforderung, dass eine eindeutige Visualisierung einer erfolgreichen physikalischen Penetration in das Stratum Corneum oder durch das Stratum Corneum in die darunterliegenden Hautschichten (Epidermis, Dermis) unter Freisetzung des Penetrationsindikators zu erfolgen hat.

Daher besteht die Aufgabe einen sicheren Nachweis einer ausreichenden Penetrationstiefe der Mikronadeln in die Haut, insbesondere in das Stratum Corneum zu ermöglichen, wobei ein direkter und unmittelbarer Nachweis möglich ist.

Die vorliegende Erfindung macht insbesondere von der Tatsache Gebrauch, dass der pH-Wert auf der Hautoberfläche 4-5,5 beträgt und im Vergleich hierzu der pH-Wert des Stratum Corneum 6,5-7 beträgt. Folglich kann mittels eines pH-Indikators beim Durchtreten von der Hautoberfläche in das Stratum Corneum ein sichtbarer Farbumschlag erzeugt werden. Weiterhin kann ebenfalls ein Farbumschlag in Abhängigkeit der Umgebung in der erlangten Penetrationstiefe erfolgen und zwar unter Berücksichtigung der intradermalen Umgebung (SC, Epidemis, Dermis), auch ohne Abhängigkeit von einem pH-Wert. Dies ist vor allem dann hilfreich, wenn dieser Farbumschlag mit dem Penetrieren von Mikronadeln in die Haut einhergeht.

Erfindungsgemäß werden solche geeigneten Stoffe Farbumschlag-Indikatoren genannt. Der Begriff Farbumschlag bedeutet, dass ein Wechsel der Farbe erfolgt, auch von farblos in nichtfarblos, welches mit dem bloßen Auge, vorzugsweise an der Hautoberfläche zu erkennen ist.

Daher betrifft die Erfindung eine solche mit den Merkmalen des Patentanspruches 1, nämlich einen Mikronadelarray zur Verwendung in der intradermalen Applikation umfassend eine Vielzahl an Mikronadeln an einem Träger, wobei die Mikronadeln mindestens einen Farbumschlag-Indikator enthalten, wobei die Mikronadeln mindestens einen Wirkstoff enthalten und die Mikronadeln mindestens ein Polymer aus biologisch abbaubaren Polymeren, biokompatiblen Polymeren oder wasserlöslichen Polymeren enthalten, wobei ein Farbumschlag oder Farbänderung eine hinreichende Penetrationstiefe in das Strateum Corneum anzeigt, wobei der Farbumschlag-Indikator Bestandteil der Mikronadeln ist, wobei der mindestens eine Farbumschlag-Indikator in die Mikronadeln eingebracht oder eingearbeitet ist.

Ein Farbumschlag, insbesondere eines pH-Indikators, zeigt eine hinreichende Penetrationstiefe in das Strateum Corneum an.

In einer weiteren Ausführungsform kann der Farbumschlag-Indikator in den Mikronadeln und im Träger enthalten sein.

Der Mikronadelarray kann eine Vielzahl von Mikronadeln aufweisen, um einen Stoff über die Haut oder in die Haut eines Patienten abgeben zu können, wobei der Mikronadelarray auf die Haut des Patienten aufgebracht wird. Jede der Mikronadeln des Mikronadelarrays weist vorzugsweise einen langgestreckten Schaft mit zwei Enden auf, das eine Ende des Schafts ist die Basis der Mikronadel, mit der die Mikronadel an dem flächigen Träger befestigt oder mit der die Mikronadel in den flächigen Träger integriert ist. Das der Basis gegenüberliegende Ende des Schafts ist vorzugsweise spitz zulaufend ausgestaltet, um ein möglichst leichtes Eindringen der Mikronadel in die Haut zu ermöglichen. Eine hohle Mikronadel kann mindestens einen Durchgang beziehungsweise Kanal oder mindestens eine Bohrung aufweisen, der/die sich von der Basis der Mikronadel bis zur Spitze der Mikronadel oder bis annähernd zur Spitze der Mikronadel erstreckt. Die Durchgänge weisen vorzugsweise einen runden Durchmesser auf.

Die Mikronadeln können massiv oder teilmassiv oder hohl ausgeführt sein.

Bevorzugte Materialien zur Herstellung solcher Mikronadeln sind biologisch abbaubare Polymere, vorzugsweise biokompatible Polymere als auch wasserlösliche Polymere, insbesondere biologischen oder nicht biologischen Ursprungs handeln, vorzugsweise Polymere, wie alpha-Hydroxysäuren, solche wie Milchsäure und/oder Glykolsäure, Polylactide, Polyglykolide, Polylactide-co-glykolide, und Copolymere mit Polyethylenglykol, Polyanhydride, Poly(ortho)ester, Polyurethane, Polybuttersäuren, Polyvaleriansäuren, und Polylactid-co-caprolactone, Polyvinylpyrrolidon (PVP), Glycane, Glycosaminglycane, Hyaluronan..

Die Mikronadeln können einen Schaft mit einem runden Querschnitt oder einen nicht runden Querschnitt aufweisen, beispielsweise mit einem dreieckigen, viereckigen oder einem polygonalen Querschnitt. Der Schaft kann einen Durchgang oder mehrere Durchgänge aufweisen, die von der Nadelbasis bis zur Nadelspitze oder annähernd zur Nadelspitze verläuft/verlaufen. Die Mikronadeln können als (Wider-)Haken ausgebildet sein, wobei ein oder mehrere dieser Mikronadeln einen oder mehrere solcher Haken aufweist. Weiterhin können die Mikronadeln schraubenförmig gestaltet und drehbar angeordnet sein und dadurch beim Anwenden einer rotierenden Bewegung das Eindringen in die Haut erleichtern und eine Verankerung in der Haut bewirken (DE 103 53 629 A1), insbesondere in der gewünschten Penetrationstiefe in der Epidermis.

Der Durchmesser einer Mikronadel beträgt üblicherweise zwischen 1 µm und 500 µm, vorzugsweise zwischen 10 µm und 100 µm. Der Durchmesser eines Durchgangs beträgt üblicherweise zwischen 3 µm und 80 µm und ist für das Durchtreten von vorzugsweise flüssigen Stoffen, Lösungen und Stoffzubereitungen geeignet. Die Länge einer Mikronadel beträgt üblicherweise zwischen 10 µm und 1.000 µm, insbesondere zwischen 100 µm und 500 µm.

Die Mikronadeln sind mit ihrer Basis an einem flächigen Träger befestigt oder in einen flächigen Träger integriert. Die Mikronadeln sind vorzugsweise im Wesentlichen senkrecht zur Fläche des Trägers stehend angeordnet. Die Mikronadeln können regelmäßig oder unregelmäßig angeordnet sein. Eine Anordnung von mehreren Mikronadeln kann Mikronadeln mit unterschiedlichen Querschnittformen, unterschiedlich dimensionierten Durchmessern und/oder unterschiedlichen Längen aufweisen. Die Anordnung aus mehreren Mikronadeln kann ausschließlich hohle Mikronadeln aufweisen. Die Anordnung kann ebenfalls massive Mikronadeln umfassen, als auch teilmassive Komposite, wie beispielsweise mit Flüssigkeitseinschlüssen durchsetzte massive Mikronadeln aufweisen.

Der Mikronadelarray kann einen flächigen Träger aufweisen, wobei der Träger im Wesentlichen eine scheibenförmige, plattenförmige oder folienförmige Grundform hat. Der Träger kann eine runde, ovale, dreieckige, viereckige oder polygonale Grundfläche haben. Der Träger kann aus unterschiedlichen Materialien hergestellt sein, beispielsweise aus einem Metall, einem keramischen Werkstoff, einem Halbleiter, einem organischen Material, einem Polymer oder einem Komposit. Als Materialien, die zur Herstellung des Trägers geeignet sind, können vorzugsweise Folien oder bahnförmige Materialien genannt werden, beispielsweise mikroporöse Membranen, vorzugsweise aus Polyethylen (PE) oder Polypropylen (PP), oder Diffusions-Membranen, vorzugsweise aus Ethylen-Vinylacetat-Copolymer (EVA) oder Polyurethan (PUR). Geeignete Materialien zur Herstellung des Trägers können aus der Gruppe ausgewählt sein, die Polyester wie Polyethylenterephthalate (PET), Polycarbonate (PC), Polyetherketone (PAEK) Polyethylennaphthalat (PEN), Polybutylenterephthalate (PBT), Polyurethane (PU), Polystyrole (PS), Polyamide (PA), Polyoxymethylen (POM), Polyolefine wie Polyethylen (PE) und Polypropylen (PP), Polytetrafluorethylen (PTFE), Polyvinylchlorid (PVC), Polyvinylidenchlorid (PVDC), Polylactat (PLA), und Kunststoffe auf Cellulose-Basis wie Cellulosehydrat oder Celluloseacetat umfasst. Geeignete Materialien zur Herstellung des Trägers können auch aus der Gruppe der Metalle ausgewählt sein, die Aluminium, Eisen, Kupfer, Gold, Silber, Platin, Legierungen der vorgenannten Metalle und andere pharmazeutisch annehmbare Metallfolien oder mit Metall bedampfte Folien umfassen.

Vorzugsweise ist der Träger aus einem flexiblen Material hergestellt, beispielsweise einem Kunststoff. Ein Träger aus einem flexiblen Material kann sich besser der Hautoberfläche und ihrer Krümmung anpassen als ein Träger aus einem nicht flexiblen Material. Dadurch wird ein besserer Kontakt des Mikronadelarrays mit der Haut erreicht, wodurch die Verlässlichkeit des Mikronadelarrays verbessert wird.

Erfindungswesentlich ist nunmehr, dass ein Farbumschlag-Indikator, insbesondere pH-Indikator, in die Mikronadeln eingebracht oder eingearbeitet ist.

Vorzugsweise weist ein pH-Indikator oder eine entsprechende Mischung an pH-Indikatoren im Bereich zwischen pH 4-8, insbesondere 4,5 ― 7 einen Farbwechsel oder Farbumschlag auf, welcher mit dem bloßen Auge sichtbar ist. Bevorzugt sind solche pH-Indikatoren, die, wenn sie auf der Haut aufliegen, eine deutlich andere Farbe anzeigen, als wenn sie in die Haut penetriert sind, insbesondere das Stratum Corneum durch Penetration erreichen. Auf diese Art und Weise kann ein deutlicher Farbumschlag oder Farbunterschied erzielt werden, der spätestens nach dem Abziehen der Mikronadeln deutlich zu ersehen ist.

Geeignete pH-Indikatoren können vorzugsweise im Bereich von pH 4-8,5 nicht abschließend sein: Nitrazingelb, Bromthymolblau, Carminsäure, Brasilin, Kongorot, Neutralrot, Phenolphthalein, Phenolrot, Tetrabromphenolphtalein, oder eine Mischung davon.

Ebenfalls können die pH-Indikatoren auch als Mischungen vorliegen und entsprechende Mischfarben können erzeugt werden.

In einer besonders bevorzugen Ausführungsform ist der pH-Indikator physiologisch akzeptabel, solche wie Nitrazingelb und Bromthymolblau.

Weiterhin kann der Farbumschlag-Indikator ausgewählt sein aus einer Verbindung, die aus Chromoionophoren oder Fluoroionophoren ausgewählt ist, wobei die Verbindung aus mindestens einem Ionophor besteht, das kovalent an mindestens ein Molekül gebunden ist, das unter den Chromophoren und Fluorophoren ausgewählt ist. Die Ionophore sind Moleküle, die Ionen zu komplexieren vermögen.

Chromoionophoren oder Fluoroionophoren verfügen über formgebenden Gruppen, die in Gegenwart der intradermalen Umgebung (SC, Epidemis, Dermis) einen Farbumschlag erzeugen können, wobei das Absorptionsspektrum dieser Moleküle durch Delokalisierung der Elektronen verändert werden kann, was zur Folge hat, dass sich die Farbe der Chromoionophoren und/oder Fluoroionophoren ändert. Die Chromoionophoren und Fluoroionophoren sind Verbindungen, die aus einem oder mehreren ionophoren Molekülen bestehen, die kovalent an mindestens ein Molekül gebunden sind, das unter den Chromophoren und Fluorophoren ausgewählt ist. Ein Chromophor ist eine Verbindung, die im sichtbaren Bereich absorbiert, d. h., die Verbindung ist farbig. Im Sinne der vorliegenden Anmeldung ist ein Fluorophor eine Verbindung, die im UV-Bereich und im sichtbaren Bereich absorbiert, diese Verbindung ist also farbig.

Geeignete Chromophore oder Fluorophore sind erfindungsgemäß solche, die unter den neutralen, sauren oder basischen nitrierten Benzolfarbstoffen, neutralen, sauren oder basischen (Di)Azofarbstoffen, einschließlich Benzidinfarbstoffen, Chinon-Farbstoffen und insbesondere neutralen, sauren oder basischen Anthrachinon-Farbstoffen, Azin-Farbstoffen, Methin-Farbstoffen, wie neutralen, sauren oder basischen Methinen und Azomethinen, Triarylmethan-Farbstoffen, Indoamin-Farbstoffen, natürlichen Farbstoffen, wie Carotinoiden, Terpenoiden, Flavonoiden, Porphyrinen, Fluorescin, Rhodamin und Cumarinen als auch jeweils Direktfarbstoffe davon ausgewählt sind.

Ein geeignetes Ionophor ist beispielsweise ein Salz (Alkalisalz), wie Na₂-, K₂HPO₄, Na, HHCO₃, Chelatbildner, Podanden, Koronanden und Kryptanden. Makrozyklen, die als Kronenether oder Koronanden bezeichnet werden, und Makrobicyclen, die Kryptanden genannt werden. Im Sinne der vorliegenden Anmeldung sind unter "Podanden" Moleküle mit offener Struktur zu verstehen, die befähigt sind, Ionen zu komplexieren und bei denen der komplexierende Bereich eine Kette ist, die Heteroatome enthält. Diese Moleküle sind beispielsweise Oligoether. Im Sinne der vorliegenden Anmeldung sind unter einem "Koronanden" zweidimensionale Moleküle mit geschlossener Struktur zu verstehen, die befähigt sind, Ionen zu komplexieren. Im Allgemeinen handelt es sich um monocyclische Moleküle auf Kohlenwasserstoffbasis, die Heteroatome enthalten. Diese Moleküle sind beispielsweise makrocyclische Polyether, die die folgende Einheit enthalten: -(CH2-CH2-Y)n-, worin Y ein Heteroatom bedeutet, das unter O, S, N und P ausgewählt ist, und n eine ganze Zahl größer 2 und vorzugsweise im Bereich von 4 bis 10 ist.

Diese Moleküle sind beispielsweise Kronenether, wie 15-Crown-5, 18-Crown-6; Benzokronenether, wie Benzo-15-crown-5, Benzo-18-crown-6, Dibenzo-15-crown-5, Dibenzo-18-crown-6; Monoazakronenether oder Diazakronenether, wie Aza-15-crown-5, Aza-18-crown-6, Diaza-15-crown-5, Diaza-18-crown-6. Im Sinne der vorliegenden Erfindung sind unter "Kryptanden" dreidimensionale Moleküle mit geschlossener Struktur (Käfige) zu verstehen, die Ionen zu komplexieren vermögen. Es handelt sich im Allgemeinen um bicyclische Moleküle auf Kohlenwasserstoffbasis, die Heteroatome enthalten. Diese Moleküle sind beispielsweise makrobicyclische Polyether, die die folgende Einheit enthalten: -(CH2-CH2-Y)n-, worin Y ein Heteroatom bedeutet, das unter O, S, N und P ausgewählt ist, und n eine ganze Zahl über 2 und vorzugsweise im Bereich von 2 bis 10 ist.

Die vorgenannten Farbumschlag-Indikatoren können ebenfalls ein Oxidationsmittel enthalten. Das Oxidationsmittel ist vorzugsweise aus Wasserstoffperoxid, Harnstoffperoxid, Salzen von Persäuren, wie Perboraten und Persulfaten, Persäuren und besonders bevorzugt aus Enzymen (Oxidasen, Peroxidasen) ausgewählt.

In einer weiteren Ausführungsform kann die Erfindung einen Farbumschlag-Indikator umfassen, der körpereigene Enzyme, insbesondere Peroxidasen nachweist, die nicht Bestandteil der Oberhaut (Epidermis) sind, beispielsweise bei Peroxiden infolge von körpereigener Bildung von Wasserstoffperoxid aus der Oxidation von Glucose mithilfe von Glucose-Oxidase, wie z.B. mittels Benzidine, insbesondere 3,3',5,5'-Tetramethylbenzidin mit spezifischer Blaufärbung sowie Phthalazine, wie 5-amino-2,3-dihydrophthalazine-1,4-dione.

Weiterhin ist ein geeigneter Farbumschlag-Indikator Tinte, insbesondere biosensing ink.

Der Farbumschlag-Indikator, insbesondere pH-Indikator, ist in den Mikronadeln oder in die Matrix oder Formulierung des Mikroarrays integriert bzw. eingearbeitet. Weiterhin kann ein Farbumschlag-Indikator auf den Mikronadeln oder auf dem Träger aufgebracht sein. Erfindungsgemäß sind lediglich solche Mikronadelarrays, bei denen ein Farbumschlag-Indikator, insbesondere pH-Indikator, Bestandteil der Mikronadel ist und hierzu in die Mikronadeln eingebracht oder eingearbeitet ist.

Insbesondere kann bei der Herstellung der Mikronadeln der Farbumschlag-Indikator zugesetzt werden, wie z.B. zu einem Polymer.

Ein erfindungsgemäßer Farbumschlag-Indikator, insbesondere pH-Indikator oder eine-Mischung kann z.B. bei der Herstellung eines solchen Mikronadelarrays beispielsweise in die Matrix hinzugegeben werden. In einer weiteren Ausführungsform ist ein solcher Farbumschlag-Indikator Bestandteil oder Inhaltsstoff eines Mikronadelarrays bzw. Mikronadeln, bzw. die Mikronadeln enthalten oder bestehen aus mindestens einem Farbumschlag-Indikator.

In einer besonders bevorzugten Ausführungsform ist z.B. Farbumschlag-Indikator in der Spitze mindestens einer Mikronadel enthalten. Beispielsweise kann der Farbumschlag-Indikator, insbesondere pH-Indikator in den Spitzen der Mikronadeln integriert sein bzw. eingearbeitet sein. Insbesondere kann bei der Herstellung der Spitzen der Mikronadeln der Farbumschlag-Indikator zugesetzt werden, wie z.B. zu einem Polymer.

In einer weiteren bevorzugten Ausführungsform kann ein Wirkstoff, insbesondere Arzneimittel in den Mikronadeln oder in die Matrix oder Formulierung des Mikroarrays integriert sein bzw. eingearbeitet sein. Weiterhin kann mindestens ein Wirkstoff, insbesondere Arzneimittel auf den Mikronadeln oder auf dem Träger aufgebracht sein. Bevorzugt ist jedoch, dass der Wirkstoff, insbesondere Arzneimittel, Bestandteil der Mikronadel ist und hierzu in die Mikronadeln eingebracht oder eingearbeitet sein kann, insbesondere in der Spitze der Mikronadeln. Insbesondere kann bei der Herstellung der Mikronadeln der Wirkstoff, insbesondere Arzneimittel zugesetzt werden, wie z.B. zu einem Polymer. Die Mikronadeln enthalten oder bestehen aus mindestens einem Arzneimittel.

Solche Arzneimittel sind bevorzugt solche wie in der EU-Richtlinie 2001/83/EG (Gemeinschaftskodex für Humanarzneimittel) definiert.

In einer weiteren besonderen Ausführungsform können ausgewählte Mikronadeln mit einem Farbumschlag-Indikator, insbesondere pH-Indikator versehen sein.

Dies erlaubt ebenfalls das vorteilhafte Aufbringen von Mustern auf der Haut, insbesondere solche Muster wie "Plus, Kreuz, Stern, Kreis" u. a. beliebige geometrische Figuren, die dem Patienten oder Anwender auch in einer visuellen grafischen Ausgestaltung die erfolgreiche Applikation bzw. Penetration der Mikronadeln mit dem bloßen Auge anzeigt.

Weiterhin ist bevorzugt, dass der Mikronadelarray oder die Mikronadeln ganz oder teilweise aus den wasserlöslichen oder biologisch abbaubaren Polymeren (supra) bestehen, so dass ein Fluidkanal die erfindungsgemäßen Farbumschlag-Indikatoren leichter freisetzen kann.

In einer weiteren Ausführungsform ist der Mikronadelarray Gegenstand eines Mikronadelsystems. Ein solches Mikronadelsystem kann mit üblichen funktionellen Gegenständen ausgestaltet sein, welche ein Fixieren auf der Haut erlauben, als auch eine leichte Handhabung zum Druckausüben auf die Haut, insbesondere mindestens ein Reservoir und einen Applikator aufweisen.

Das Mikronadelsystem kann mindestens ein Reservoir aufweisen, welches mindestens einen beliebigen Stoff, insbesondere Wirkstoff, Hilfsstoff oder Arzneistoff vorzugsweise in Form einer Lösung oder Zubereitung enthält.

Das Reservoir dient der Aufbewahrung des im System enthaltenen mindestens einen beliebigen Stoff, Wirkstoff oder Arzneistoff.

Das Reservoir steht mit den Durchgängen der hohlen Mikronadeln derart in Verbindung, dass ein Flüssigkeitsanschluss zwischen dem Reservoir und den Durchgängen der mit dem Reservoir in Verbindung stehenden Mikronadeln besteht. Dadurch kann der Inhalt des Reservoirs über die Durchgänge der Mikronadeln aus dem Reservoir heraus aus dem Mikronadelsystem freigesetzt werden, wenn nach Applikation des Mikronadelsystems auf die Haut eines Patienten Druck auf das Reservoir ausgeübt wird. Die vorrätige Zubereitung tritt an oder in der Nähe der Spitzen der Mikronadeln aus dem Mikronadelsystem aus und kann in das Zielgewebe eindringen. Üblicherweise ist das Reservoir an einer Oberfläche des flächigen Trägers befestigt, nämlich an der Oberfläche des Trägers, die der Oberfläche des Trägers gegenüberliegt, aus der die Mikronadeln herausragen.

Das Reservoir ist leicht komprimierbar, um dem auf das Reservoir ausgeübten Druck wenig Widerstand entgegenzubringen und somit den Druck auf die in dem Reservoir enthaltene Zubereitung zum Austreten weiterleiten zu können. Gemäß einer Ausführungsform kann das Reservoir beispielsweise in Form eines flexiblen Beutels vorliegen.

Gemäß einer bevorzugten Ausführungsform ist das Reservoir als Kissen oder Ballon ausgestaltet und aus einem elastischen Material hergestellt, beispielsweise aus einem elastomeren Polymer oder Gummi. Beispiele für Polymere sind Polyester wie Polyethylenterephthalate (PET), Polycarbonate (PC), Polyetherketone (PAEK), Polvethylennaphthalat (PEN), Polvbutvlenterephthalate (PBT), die Polyurethane (PU), Polystyrole (PS), Polyamide (PA), Polyoxymethylen (POM), Polyolefine wie Polyethylen (PE) und Polypropylen (PP), Polytetrafluorethylen (PTFE), Polyvinylchlorid (PVC), Polyvinylidenchlorid (PVDC). Polylactat (PLA), und Kunststoffe auf Cellulose- Basis wie Cellulosehydrat oder Celluloseacetat.

In einer weiteren bevorzugten Ausführungsform ist das Mikronadelsystem enthaltend einen Mikronadelarray mit einem Applikator ausgestaltet. Solche Applikatoren erlauben vorteilhaft die Aktivierung eines Druckmechanismus zur Penetration des Mikronadelarrays in die Haut bzw. Stratum Corneum (siehe z.B. WO2008091602A2, WO2016162449A1).

Gemäß einer weiteren Ausführungsform kann der Mikronadelarray Fixiermittel enthalten, die vorzugsweise mit Hilfe eines haftklebenden Klebestreifens oder Pflasters auf der Haut eines Patienten oder Probanden befestigt werden. Als Haftkleber eignen sich hochviskose Stoffe, die nach kurzem leichtem Druck auf der Haut kleben, so genannte druckempfindliche Haftkleber (PSA). Sie besitzen hohe Kohäsions- und Adhäsionskräfte. Zum Einsatz kommen können beispielsweise Haftkleber auf Basis von Poly(meth)acrylaten, auf Basis von Polyisobutylenen oder auf Basis von Silikonen.

Daher betrifft die Erfindung einen erfindungsgemäßen Mikronadelarray zur intradermalen Applikation, der Fixiermittel für die Haut aufweist.

Der Begriff "intradermale Applikation (synonym: "intrakutane Applikation") beschreibt erfindungsgemäß die Verabreichung von beliebigen Stoffen über den Mikronadelarray in die Haut und erfordert ein Einstechen der Mikronadeln in die Haut.

Ebenfalls offenbart, jedoch nicht Teil der beanspruchten Erfindung, ist ein Verfahren zur intradermalen Applikation, wobei mindestens ein Stoff mittels eines Mikronadelarrays umfassend Fixiermittel für die Haut und eine Vielzahl an Mikronadeln an einem Träger appliziert wird, wobei die Mikronadeln und / oder der Träger einen pH-Indikator enthalten.

Nachfolgende Beispiele dienen zur weiteren Erörterung der Erfindung, ohne jedoch die Erfindung auf diese Beispiele zu beschränken.

### Beispiel 1: Nachweis der Funktionalität

Verwendung von Nitrazingelb (pH-Indikator) für die Applikation mittels Mikronadeln. Nach erfolgreicher Applikation ist eine Blaufärbung nach ca. 10 Sekunden mit dem bloßen Auge sichtbar. Der unterschiedliche pH-Wert auf der Hautoberfläche pH 4-5,5 im Vergleich zum pH-Wert des Stratum Corneum pH 6,5-7 erlaubt eine differenzierte Bewertung der Penetration,
a) Blaufärbung heißt erfolgreiche Applikation, d. h. Penetration der Mikronadeln in das Stratum Corneum,
b) Haut bleibt unverändert bis schwach gelblich verfärbt, d. h. keine erfolgreiche Penetration der Mikronadeln in die das Stratum Corneum.

In diesem Ausführungsbeispiel wurden als Indikator geringste Mengen an Nitrazingelb (0,02%) in die Matrix der Mikronadeln hinzugegeben.

### Beispiel 2:

Als weiteres Ausführungsbeispiel kann die Verwendung von Farbumschlag-Indikatoren genannt werden, welche die Gegenwart eines in der Haut vorhandenen Stoffes nutzt, welcher im Gegensatz dazu auf der Oberhaut (Epidermis) nicht vorhandenen ist.

Ein solcher Farbumschlag-Indikator kann aus einer oder mehreren stofflichen Komponenten bestehen. Gegenwart und Abwesenheit eines Stoffes in der Haut sind hierbei als relative Angabe zu verstehen und müssen, mit Bezug auf die erfolgreiche Penetration in die Haut, einen für den Indikator ausreichendes Unterscheidungsmerkmal erreichen.

Insbesondere aber nicht abschließend bietet sich im Sinne der Erfindung die Verwendung eines Glukose-sensitiven Indikators an. Hierbei kann die für den Nachweis von Glukose in menschlichen Körperflüssigkeiten bekannte Reaktion genutzt werden, bei welcher die Oxidation von Glucose durch Glucose-Oxidase, sowie eine nachfolgende durch Peroxidase katalysierte Reaktion der Umsetzungsprodukte, zum Farbumschlag einer stofflichen Teilkomponente des Indikators führt.

Geeignete Stoffe zur differenzierten Bewertung eines Farbumschlags bei erfolgter Penetration in die Haut sind dabei vorzugsweise aber nicht abschließend 3,3',5,5'-Tetramethylbenzidin (TMB/TMBH2) und von der Strukturklasse der Benzidine abgeleitete Derivate, sowie Phthalazine, wie 5-amino-2,3-dihydrophthalazine-1,4-dione und davon abgeleitete Derivate.

Die differenzierte Bewertung der Penetration wird bei Verwendung der Leukoform von TMB bzw. TMBH2 als Teilkomponente des Indikators entsprechend ermöglicht durch:
a) Blaufärbung - heißt erfolgreiche Applikation, d. h. Penetration der Mikronadeln in das Stratum Corneum. Die Farbveränderung von TMBH2 (farblos) zu TMB (blau) basiert auf der oben beschriebenen Reaktion des Indikators.
b) Haut bleibt unverändert, bedeutet keine erfolgreiche Penetration der Mikronadeln in die das Stratum Corneum und entsprechendes Ausbleiben der Reaktion der Indikatorstoffe.

## Patentansprüche

1. Mikronadelarray zur Verwendung in der intradermalen Applikation umfassend eine Vielzahl an Mikronadeln an einem Träger, **dadurch gekennzeichnet, dass** die Mikronadeln mindestens einen Farbumschlag-Indikator enthalten, wobei die Mikronadeln mindestens einen Wirkstoff enthalten und die Mikronadeln mindestens ein Polymer aus biologisch abbaubaren Polymeren, biokompatiblen Polymeren oder wasserlöslichen Polymeren enthalten, wobei ein Farbumschlag oder Farbänderung eine hinreichende Penetrationstiefe in das Strateum Corneum anzeigt, **dadurch gekennzeichnet, dass** der Farbumschlag-Indikator Bestandteil der Mikronadeln ist. wobei der mindestens eine Farbumschlag-Indikator in die Mikronadeln eingebracht oder eingearbeitet ist.

2. Mikronadelarray zur Verwendung nach Anspruch 1, **dadurch gekennzeichnet, dass** zur Herstellung der Mikronadeln der Farbumschlag-Indikator zugesetzt wird.

3. Mikronadelarray zur Verwendung nach Anspruch 1 oder Anspruch 2, **dadurch gekennzeichnet, dass** die Mikronadeln mindestens einen Wirkstoff, und zwar ein Arzneimittel enthalten.

4. Mikronadelarray zur Verwendung nach Anspruch 1 oder Anspruch 2, **dadurch gekennzeichnet, dass** der Farbumschlag-Indikator mindestens ein pH-Indikator ist, insbesondere ausgewählt aus der Gruppe Nitrazingelb, Bromthymolblau, Carminsäure, Brasilin, Kongorot, Neutralrot, Phenolphthalein, Phenolrot, Tetrabromphenolphtalein, oder eine Mischung davon oder eine Mischung davon.

5. Mikronadelarray zur Verwendung nach Anspruch 4, **dadurch gekennzeichnet, dass** der Farbumschlag-Indikator mindestens ein physiologisch akzeptabler pH-Indikator ist, insbesondere Nitrazingelb oder Bromthymolblau.

6. Mikronadelarray nach einem der Ansprüche 1 bis 3, **dadurch gekennzeichnet, dass** der Farbumschlag-Indikator eine Verbindung, die aus Chromoionophoren oder Fluoroionophoren ausgewählt ist, wobei die Verbindung aus mindestens einem lonophor besteht, dass kovalent an mindestens ein Molekül gebunden ist, dass aus Chromophoren oder Fluorophoren ausgewählt ist.

7. Mikronadelarray zur Verwendung nach Anspruch 6, **dadurch gekennzeichnet, dass** die Chromophoren oder Fluorophoren aus der Gruppe ausgewählt sind aus den neutralen, sauren oder basischen nitrierten Benzolfarbstoffen, neutralen, sauren oder basischen Azofarbstoffen, Chinon-Farbstoffen und insbesondere neutralen, sauren oder basischen Anthrachinon-Farbstoffen, Azin-Farbstoffen, Methin-Farbstoffen, wie neutralen, sauren oder basischen Methinen und Azomethinen, Triarylmethan-Farbstoffen, Indoamin-Farbstoffen, natürlichen Farbstoffen, wie Carotinoiden, Terpenoiden, Flavonoiden, Porphyrinen, Fluorescin, Rhodamin und Cumarinen als auch jeweils Direktfarbstoffe davon.

8. Mikronadelarray nach einem der vorstehenden Ansprüche, **dadurch gekennzeichnet, dass** der Farbumschlag-Indikator Tinte, insbesondere biosensing ink ist.

9. Mikronadelarray nach einem der Ansprüche 1 bis 3, **dadurch gekennzeichnet, dass** der Farbumschlag-Indikator körpereigene Enzyme, insbesondere Peroxidasen nachweist, die nicht Bestandteil der Epidermis sind, insbesondere mittels Benzidinen, insbesondere 3,3',5,5'- Tetramethylbenzidin oder Phthalazine, insbesondere 5-amino-2,3-dihydrophthalazine-1,4-dione.

10. Mikronadelarray zur Verwendung nach einem der vorstehenden Ansprüche, **dadurch gekennzeichnet, dass** der Farbumschlag-Indikator in der Spitze der Mikronadeln enthalten ist.

11. Mikronadelarray zur Verwendung nach einem der vorstehenden Ansprüche, **dadurch gekennzeichnet, dass** Muster, insbesondere solche wie "Plus, Kreuz, Stern, Kreis" oder andere beliebige geometrische Figuren mit dem bloßen Auge auf der Haut zu erkennen sind.

12. Mikronadelarray zur Verwendung nach einem der vorstehenden Ansprüche, **dadurch gekennzeichnet, dass** Mikronadeln bestehend aus biologisch abbaubaren Polymeren, biokompatiblen Polymeren oder wasserlöslichen Polymeren verwendet werden, und zwar Polymere von alpha-Hydroxysäuren, solche wie Milchsäure und/oder Glykolsäure, Polylactide, Polyglykolide, Polylactide-co-glykolide, und Copolymere mit Polyethylenglykol, Polyanhydride, Poly(ortho)ester, Polyurethane, Polybuttersäuren, Polyvaleriansäuren, und Polylactid-co-caprolactone, Polyvinylpyrrolidon (PVP), Glycane, Glycosaminglycane, Hyaluronan.

13. Mikronadelarray zur Verwendung nach einem der vorstehenden Ansprüche, **dadurch gekennzeichnet, dass** der Mikronadelarray Fixiermittel aufweist, insbesondere ein Klebestreifen oder Pflaster.

14. Mikronadelarray zur Verwendung nach einem der vorstehenden Ansprüche, wobei der Mikronadelarray in einem Mikronadelsystem aufgenommen ist und mindestens ein Reservoir und / oder Applikator enthält.

## Claims

1. A microneedle array for use with intradermal delivery, comprising a plurality of microneedles on a carrier, **characterized in that** the microneedles comprise at least one color change indicator, the microneedles comprising at least one active ingredient and the microneedles comprising at least one polymer made of biodegradable polymers, biocompatible polymers or water-soluble polymers, a color change or color shift indicating a sufficient penetration depth into the *stratum corneum,* **characterized in that** the color change indicator is an integral part of the microneedles, the at least one color change indicator being introduced or incorporated into the microneedles.

2. The microneedle array for use according to claim 1, **characterized in that** the color change indicator is added during the production of the microneedles.

3. The microneedle array for use according to claim 1 or claim 2, **characterized in that** the microneedles comprise at least one active ingredient, and more particularly a medicinal drug.

4. The microneedle array for use according to claim 1 or claim 2, **characterized in that** the color change indicator is at least one pH indicator, in particular selected from the group consisting of nitrazine yellow, bromothymol blue, carminic acid, brazilin, Congo red, neutral red, phenolphthalein, phenolic red, tetrabromophenolphthalein or a mixture thereof or a mixture thereof.

5. The microneedle array for use according to claim 4, **characterized in that** the color change indicator is at least one physiologically acceptable pH indicator, and in particular nitrazine yellow or bromothymol blue.

6. The microneedle array according to any one of claims 1 to 3, **characterized in that** the color change indicator is a compound selected from chromoionophores or fluoroionophores, the compound being composed of at least one ionophore covalently bound to at least one molecule, which is selected from chromophores and fluorophores.

7. The microneedle array for use according to claim 6, **characterized in that** the chromophores or fluorophores are selected from the neutral, acidic or basic nitrated benzene dyes, neutral, acidic or basic azo dyes, quinone dyes, and in particular neutral, acidic or basic anthraquinone dyes, azine dyes, methine dyes, such as neutral, acidic or basic methines and azomethines, triarylmethane dyes, indoamine dyes, natural dyes, such as carotinoids, terpenoids, flavonoids, porphyrins, fluorescein, rhodamine and coumarins, and direct dyes thereof.

8. The microneedle array according to any one of the preceding claims, **characterized in that** the color change indicator is ink, and in particular biosensing ink.

9. The microneedle array according to any one of claims 1 to 3, **characterized in that** the color change indicator detects endogenous enzymes, and in particular peroxidases, which do not form part of the epidermis, in particular by means of benzidines, in particular 3,3',5,5'-tetramethylbenzidine, or phthalazines, in particular 5-amino-2,3-dihydrophthalazine-1,4-di one.

10. The microneedle array for use according to any one of the preceding claims, **characterized in that** the color change indicator is included in the tip of the microneedles.

11. The microneedle array for use according to any one of the preceding claims, **characterized in that** patterns, and in particular those such as "plus, cross, star, circle" or other arbitrary geometric figures are visible on the skin to the naked eye.

12. The microneedle array for use according to any one of the preceding claims, **characterized in that** microneedles made of biodegradable polymers, biocompatible polymers or water-soluble polymers are used, and more particularly polymers of alpha-hydroxy acids, such as lactic acid and/or glycolic acid, polylactides, polyglycolides, polylactide-co-glycolides, and copolymers with polyethylene glycol, polyanhydrides, poly(ortho)esters, polyurethanes, polybutyric acids, polyvaleric acids, and polylactide-co-caprolactones, polyvinyl pyrrolidone (PVP), glycans, glycosaminoglycans and hyaluronan.

13. The microneedle array for use according to any one of the preceding claims, **characterized in that** the microneedle array comprises fixation means, and in particular adhesive strips or patches.

14. The microneedle array for use according to any one of the preceding claims, wherein the microneedle array is accommodated in a microneedle system and comprises at least one reservoir and/or applicator.

## Revendications

1. Matrice de micro-aiguilles destinée à l'utilisation dans l'application intradermale comprenant une multiplicité de micro-aiguilles sur un support, **caractérisée en ce que** les micro-aiguilles contiennent au moins un indicateur de changement de couleur, où les micro-aiguilles contiennent au moins une substance active et les micro-aiguilles contiennent au moins un polymère à base de polymères biodégradables, de polymères biocompatibles ou de polymères solubles dans l'eau, où un changement de couleur ou une modification de couleur indique une profondeur de pénétration atteignant la stratum corneum, **caractérisée en ce que** l'indicateur de changement de couleur est un composant des micro-aiguilles, où l'au moins un indicateur de changement de couleur est incorporé ou intégré dans les micro-aiguilles.

2. Matrice de micro-aiguilles destinée à l'utilisation selon la revendication 1, **caractérisée en ce que** l'indicateur de changement de couleur est ajouté pour la fabrication des micro-aiguilles.

3. Matrice de micro-aiguilles destinée à l'utilisation selon la revendication 1 ou la revendication 2, **caractérisée en ce que** les micro-aiguilles contiennent au moins une substance active, et en l'occurrence, un médicament.

4. Matrice de micro-aiguilles destinée à l'utilisation selon la revendication 1 ou la revendication 2, **caractérisée en ce que** l'indicateur de changement de couleur est au moins un indicateur de pH, en particulier, choisi dans le groupe du jaune de nitrazine, du bleu de bromothymol, de l'acide carminique, de la brasiline, du rouge Congo, du rouge neutre, de la phénolphtaléine, du rouge de phénol, de la tétrabromo phénolphtaléine, ou un mélange de ceux-ci, ou un mélange de ceux-ci.

5. Matrice de micro-aiguilles destinée à l'utilisation selon la revendication 4, **caractérisée en ce que** l'indicateur de changement de couleur est au moins un indicateur de pH physiologiquement acceptable, en particulier, du jaune de nitrazine ou du bleu de bromothymol.

6. Matrice de micro-aiguilles selon l'une des revendications 1 à 3, **caractérisée en ce que** l'indicateur de changement de couleur est un composé qui est choisi parmi des chromo ionophores ou des fluoro ionophores, où le composé est constitué d'au moins un ionophore qui est relié de manière covalente avec au moins une molécule qui est choisie parmi des chromophores ou des fluorophores.

7. Matrice de micro-aiguilles destinée à l'utilisation selon la revendication 6, **caractérisée en ce que** les chromophores ou les fluorophores sont choisis dans le groupe à base des colorants benzéniques nitrés neutres, acides ou basiques, des colorants azoïques neutres, acides ou basiques, de colorants de type quinone et en particulier, de colorants d'anthraquinone neutres, acides ou basiques, de colorants type azine, de colorants de type méthine, comme des méthines et azométhines neutres, acides ou basiques, de colorants de type triarylméthane, de colorants de type indoamine, de colorants naturels, comme les caroténoïdes, les terpènoïdes, les flavonoïdes, les porphyrines, la fluorescéine, la rhodamine et les coumarines, ainsi qu'également des colorants directs.

8. Matrice de micro-aiguilles selon l'une des revendications précédentes, **caractérisée en ce que** l'indicateur de changement de couleur est de l'encre, en particulier de l'encre biosensitive.

9. Matrice de micro-aiguilles selon l'une des revendications 1 à 3, **caractérisée en ce que** l'indicateur de changement de couleur se **caractérise par** des enzymes propres au corps, notamment des peroxydases qui ne sont pas des composantes de l'épiderme, notamment au moyen de benzidines, en particulier la 3, 3', 5, 5'-tétraméthyl benzidine ou de phtalazines, en particulier la 5-amino-2, 3-dihydro phtalazine-1, 4-dione.

10. Matrice de micro-aiguilles destinée l'utilisation selon l'une des revendications précédentes, **caractérisée en ce que** l'indicateur de changement de couleur est contenu dans la pointe des micro-aiguilles.

11. Matrice de micro-aiguilles destinée l'utilisation selon l'une des revendications précédentes, **caractérisée en ce que** le motif, en particulier des motifs comme « plus, croix, étoile, cercle » ou toute autre figure géométrique sont reconnaissables à l'œil nu sur la peau.

12. Matrice de micro-aiguilles destinée l'utilisation selon l'une des revendications précédentes, **caractérisée en ce que** des micro-aiguilles constituées de polymère biodégradables, de polymères biocompatibles ou de polymères solubles sont employés et en l'occurrence, des polymères d'acides alpha hydroxyles, tels que l'acide lactique et/ou l'acide glycolique, des polylactides, des polyglycolides, des polylactide-co-glycolides, et des copolymères avec du polyéthylène glycol, des poly anhydrides, des poly(ortho) esters, des polyuréthanes, des acides polybutiriques, des acides polyvalérianiques et des polylactid-co-caprolactones, de la polyvinyl pyrrolidone (PVP), des glycanes, des glycosamine glycanes, de l'hyaluronane.

13. Matrice de micro-aiguilles destinée l'utilisation selon l'une des revendications précédentes, **caractérisée en ce que** la matrice de micro-aiguilles présentent des agents de fixation, en particulier une bande adhésive ou du sparadrap.

14. Matrice de micro-aiguilles destinée l'utilisation selon l'une des revendications précédentes, **caractérisée en ce que** la matrice de micro-aiguilles est logée dans un système de micro-aiguilles et contient au moins un réservoir et/ou un applicateur.
